(19) Europäisches Patentamt / European Patent Office / Office européen des brevets

(11) **EP 2 642 290 A1**

(12) **EUROPEAN PATENT APPLICATION**

(43) Date of publication:
**25.09.2013 Bulletin 2013/39**

(51) Int Cl.:
***G01N 33/50*** (2006.01)   ***G01N 33/68*** (2006.01)

(21) Application number: **12160140.5**

(22) Date of filing: **19.03.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventor: **The designation of the inventor has not yet been filed** |
| | (74) Representative: **van den Broek, Ludovicus A.G.M. et al**<br>**Synthon B.V.**<br>**IP Research**<br>**P.O. Box 7071**<br>**6503 GN Nijmegen (NL)** |
| (71) Applicant: **Synthon BV**<br>**6545 CM Nijmegen (NL)** | |

(54) **Glatiramer acetate human monocytic cell line-based potency assay**

(57)   The present invention relates to a method to determine the potency of a batch of glatiramer acetate comprising stimulating human monocytic cell line cells with an effective amount of phorbol 12-myristate 13-acetate and lipopolysaccharide, exposing said cells to said batch of glatiramer acetate, and determining the expression of a monocyte anti-inflammatory cytokine induced by glatiramer acetate. It further relates to a process for preparing a batch of glatiramer acetate which is acceptable for pharmaceutical use, in particular for the treatment of multiple sclerosis, comprising a) preparing a batch of glatiramer acetate, b) measuring the relative potency of said batch according to the afore-mentioned method, and c) qualifying the batch as acceptable for pharmaceutical use if the relative potency so measured is between 70% and 141% of a reference batch of glatiramer acetate.

Figure 1

EP 2 642 290 A1

**Description**

The present invention relates to a method to determine the potency of a batch of glatiramer acetate.

**BACKGROUND OF THE INVENTION**

Inflammation is a hallmark of multiple sclerosis (MS) which leads to demyelination and axonal loss resulting in neurodegeneration. Although it was sometimes claimed that neurodegeneration may be independent of inflammation, recent neuropathological studies provide clear evidence that whenever active tissue destruction is seen in MS, it occurs on the background of inflammation. It has been assumed for a long time that the pro-inflammatory cytokines tumor necrosis factor (TNF) and interleukin-1$\beta$ (IL-1$\beta$) play an important part in MS progression and severity. To restrain inflammation in normal physiology, pro-inflammatory reactions are closely interconnected with counter-regulatory anti-inflammatory pathways. IL-1$\beta$ activity is restrained by several molecules including the decoy IL-1 receptor II (IL-1RII) and its soluble form and the secreted form of IL-1 receptor antagonist (sIL-1Ra) which binds IL-1RI without triggering signaling. Both IL-1$\beta$ and sIL-1Ra are mainly produced by monocytes/macrophages, which, together with T lymphocytes, are important parts of cellular infiltrate in the central nervous system (CNS) of MS patients.

Evidence of IL-1 system involvement in MS, although abundant, remains indirect. A combination of polymorphisms in the IL-1$\beta$ (IL1B) and sIL-1Ra (IL-1RN) genes has been correlated with MS disease severity. Higher in vitro sIL-1Ra production has been observed in carriers of IL-1RN allele 2, with an indication of an allelic dose-effect relationship. In a study including 377 MS patients, significant associations between IL-1 genotypes and clinical outcome were found. The same trends were subsequently demonstrated in a second independent group of 67 primary progressive MS patients, suggesting that genetically determined immunomodulation mediated by IL-1 influences long-term prognosis in MS. Families displaying high IL-1$\beta$/sIL-1Ra production ratio are at increased risk to have a relative with relapse-onset MS than families with a low ratio. Furthermore, IL-1$\beta$ is expressed throughout the CNS particularly in inflamed lesion, and caspase-1 that is required for the processing of pro-IL-1$\beta$ into active IL-1$\beta$ is expressed MS plaques. Direct evidence of IL-1 system involvement was demonstrated in the animal model for MS: Experimental Autoimmune Encephalomyelitis (EAE). Indeed, mice KO for both IL-1$\alpha$ and IL-1$\beta$ display resistance to EAE induction and reduced disease severity whereas EAE was induced in IL-1Ra KO mice in the absence of pertussis toxin. Last but not least, treatment of EAE animals with recombinant sIL-1Ra reduced disease severity. More recently, because of the importance of IL-1$\beta$ in the polarization of TH17 T cells, the inhibition of IL-1$\beta$ together with that of IL-23 by a MEK/ERK inhibitor was shown to dampen EAE severity.

Monocytes/macrophages play an important part in the pathogenesis of MS. Although the composition of the inflammatory infiltrate in the CNS varies depending on the type, stage and activity of MS, monocytes/macrophages are thought to be key effectors responsible for tissue damage. They predominate in active MS lesions, and the presence of myelin degradation products inside macrophages is one of the most reliable markers of lesion activity, and pro-inflammatory mediators of activated monocytes/macrophages contribute to myelin injury. Although pro-inflammatory cytokines are involved in destructive mechanisms, they may also participate in repair, e.g., TNF promotes proliferation of oligodendrocyte progenitors and remyelination.

Glatiramer acetate (GA) is a copolymer used as an immunomodulatory treatment in relapsing-remitting multiple sclerosis (RRMS). Although in vitro studies demonstrated GA to affect multiple target cells, its mechanisms of action are poorly understood. It has previously been shown that GA induces the production of the secreted form of IL-1 receptor antagonist (sIL-1Ra) in human monocytes and, in turn, enhances sIL-1Ra circulating levels in MS patients. See D. Burger et al. in PNAS, 2009, Vol. 106, No. 11, pages 4355-4359). Thus, IFN$\beta$ and GA, both immunomodulators used with comparable efficiency in MS therapy, induce the production of the IL-1$\beta$ inhibitor, sIL-1Ra, in monocytes in vitro and enhance sIL-IRa circulating levels in vivo. The ability of circulating sIL-1Ra to cross the blood-brain barrier indicates that it may inhibit the pro-inflammatory activities of IL-1$\beta$ into the CNS, a mechanism particularly important in regard to GA whose high polarity and hydrophilic nature is likely to impede CNS penetration. Therefore, sIL-1Ra might mediate part of the beneficial anti-inflammatory effects of GA at the periphery and into the CNS.

Glatiramer acetate (GA, COPAXONE®: 20 mg/ml GA) was FDA approved in 2002 for the treatment of relapsing forms of MS, including RRMS. GA is a copolymer and consists of the acetate salts of synthetic polypeptides made up of the naturally occurring amino acids glutamic acid, lysine, alanine, and tyrosine in specific molar ratios. To this end, see WO 95/31990 and US 3849550 cited therein. Its activity or potency is conventionally tested in an experimental animal model, notably the experimental autoimmune encephalomyelitis (EAE) mouse model. Typically, the potency of a test batch of GA is compared with a reference batch of GA. These animal studies however are elaborate, expensive, and use large numbers of test animals, which experience significant discomfort levels, and there is therefore a need for a faster and cheaper potency test which does not require test animals.

WO 03/048735 describes a first improvement over the conventional EAE mouse in vivo model. It relates to an ex-vivo mouse lymph node cell-based potency test, determining the amount of the cytokine IL-2 secreted by said cells, but it still requires immunizing female mice with GA, sacrificing mice after immunization, and is directed to T cells instead of monocytic cells.

WO 2008/157697 discloses a method for testing an amino acid copolymer by simultaneously exposing cells to the copolymer in combination with a (proinflammatory) cytokine, and determining the expression of a protein induced by said cytokine. The amino acid copolymer can be glatiramer acetate. Examples of suitable cells include (myeloid) cells such as human acute monocytic leukemia cells (THP-1), human leukemic monocyte lymphoma cells (U937), and human promyelocytic leukemia cells (HL-60). Examples of suitable (proinflammatory) cytokines include tumor necrosis factor alpha (TNF$\alpha$), interferon gamma (IFN$\gamma$), interleukin-1 beta (IL-1$\beta$), interleukin-6 (IL-6), and interleukin-8 (IL-8). Examples of proteins regulated by said cytokines include $\gamma$-interferon-inducible protein 10 (IP-10, CXCL10), interferon-inducible T cell $\alpha$-chemoattractant (I-TAC, CXCL11), and monokine induced by $\gamma$-interferon (MIG, CXCL9). Table 3 shows the GA dose dependence of IFN $\gamma$ mediated induction of IP-10, I-TAC, and MIG in TPH-1 cells and the description above said table (on page 23) mentions that the assay can be used to compare two or more copolymer preparations. However, the presented data cannot be fitted using a linear, or a non-linear four-parameter logistic model, as described in the draft USP chapter <1034> on analysis of biological assays, including cell based potency assays. These data are thus not suitable to quantitatively determine the potency of a GA test batch relative to a reference batch of GA (see the Example below). Hence, there is a need for an improved cell-based potency assay for GA, which can be routinely used to quantitatively determine the relative potency of a batch of GA as compared to a reference batch of GA.

## DETAILED DESCRIPTION OF THE PRESENT INVENTION

The present invention relates to a method to determine the potency of a batch of glatiramer acetate comprising stimulating human monocytic cell line cells with an effective amount of phorbol 12-myristate 13-acetate (PMA) and lipopolysaccharide (LPS), exposing said cells to said batch of glatiramer acetate (GA), and determining the expression of a monocyte anti-inflammatory cytokine induced by glatiramer acetate.

In one embodiment of the present invention, the method further comprises determining the relative potency by comparing the potency of a batch of GA with the potency of a reference batch of GA.

An important advantage of the present invention method is that well-defined human monocytic cell line cells are used, which allows for highly reproducible test results. It further allows quantification of relative potency between batches. It is faster and cheaper, since it does not involve testing on animals, and it is clinically relevant since it looks at the expression of an anti-inflammatory cytokine. In one particular embodiment of the present invention, the expression of a cytokine with known effect in MS is determined.

In the context of the present invention, with glatiramer acetate (GA) is meant any synthetic polypeptide or copolymer made up of the amino acids tyrosine (Y), glutamic acid (E), alanine (A), and lysine (K) in all molar ratios as well as known variations thereof made up of the amino acids tyrosine (Y), phenylalanine (F), alanine (A), and lysine (K), and salts thereof, in particular acetate salts thereof. See the paragraph starting with "Glatiramer acetate" bridging pages 1 and 2 of WO 03/048735 and the definitions of "amino acid copolymer" on pages 6 and 7 of WO 2008/157697, as well as WO 95/31990 and US 3849550 cited therein.

In the context of the present invention, with a monocyte anti-inflammatory cytokine is meant any signaling molecule that is produced by any cell of the monocytic lineage and exerts inhibitory (anti-inflammatory) effects on other cells of the immune system.

Figure 1 shows four-parameter fits of 4 replicate sIL-1Ra dose-response curves in THP-1 cells using Synthon glatiramer acetate reference material.

Figure 2 depicts the dose-response curve as derived from the data on IP-10 shown in Table 3 of WO 2008/157697.

Particularly useful human monocytic cell line cells are human acute monocytic leukemia cells (THP-1), human leukemic monocyte lymphoma cells (U937), U937 cells differentiated into microglial cells by serial treatment with PMA and interferon gamma (IFN$\gamma$), and human monocytic leukemia cells Mono Mac 6 (MM6). In a preferred embodiment of the present invention, human acute monocytic leukemia cells (THP-1), human leukemic monocyte lymphoma cells (U937) or human monocytic leukemia cells Mono Mac 6 (MM6) are used. In a more preferred embodiment, THP-1 cells are used.

Human monocytic cells are brought into a suitable medium, e.g. RPMI 1640 medium, and are stimulated with an effective amount of PMA and LPS, contacted with GA (0-250 $\mu$g/ml) and the effect on the expression of a monocyte anti-inflammatory cytokine, such as sIL-1Ra, is measured. The present inventors surprisingly have found that the cells need to be stimulated (simultaneously) with a combination of PMA and LPS, and that neither PMA nor LPS treatment of THP-1 cells alone results in GA-induced cytokine expression. Dose-response curves are generated by GA activation of monocytic cells and EC50 values are calculated using a non-linear four-parameter logistic model on semi-log transformed data, as described in the draft USP chapter <1034> on analysis of biological assays.

In accordance with the method of the present invention an effective amount of PMA and LPS should be used, and such amounts may be different for different human monocytic cell line cells. Optimal effective amounts can easily be found by one skilled in the art using the amounts described in this specification as guidance. In one embodiment of the present invention, human monocytic cells, in particular THP-1 cells, are stimulated with a combination of 5-10 ng/ml PMA and 5-25 ng/ml LPS. In a preferred embodiment, cells, in particular THP-1 cells are stimulated with a combination of 5 ng/ml PMA and 25 ng/ml LPS.

In one embodiment of the present invention, the monocyte anti-inflammatory cytokine is selected from the group consisting of secreted form of IL-1 receptor antagonist (sIL-IRa), interleukin-10 (IL-10), and transforming growth factor beta (TGFβ). In another embodiment, the monocyte anti-inflammatory cytokine is sIL-1Ra.

The present invention also relates to a process for preparing a batch of glatiramer acetate (GA) which is acceptable for pharmaceutical use comprising a) preparing a batch of glatiramer acetate, b) measuring the relative potency of said batch according to the method described above, and c) qualifying the batch as acceptable for pharmaceutical use if the relative potency so measured is between 70% and 141%, more specifically between 80% and 125% of the reference batch of glatiramer acetate.

In one embodiment of the present invention, said pharmaceutical use is for the treatment of multiple sclerosis (MS), in particular relapsing forms of MS, including relapsing-remitting MS (RRMS).

The present invention is illustrated by the following examples.

## EXAMPLES

## Example 1

### Thawing of THP-1 cells

- Prewarm 10 ml RPMI-1640 Medium with 10% FBS (fetal bovine serum) and 2 mM L-Glutamine in a 15 ml Falcon tube, prior to use in a 37°C water bath;
- Remove one vial of THP-1 cells from the liquid nitrogen storage tank and thaw the cells in the vial by gentle agitation in a 37°C $\pm$1°C water bath until almost totally thawed;
- Sterilize the outside of the vial using a wipe, soaked in 70 % (v/v) ethanol;
- Using good aseptic technique, open the vial and transfer the contents of the vial into a Falcon tube, adding 10 ml of the pre-warmed RPMI-1640 + 10% FBS and 2 mM L-Glutamine drop by drop;
- Spin down at approximately 200g for 5 minutes. Afterwards, discard the supernatant;
- Resuspend the pellet into 10 ml of pre-warmed RPMI-1640 + 10% FBS, and transfer the cell suspension to a T-75 culture flask;
- Take a sample for cell count using the Invitrogen Countess Automated cell counter;
- Place the T-75 cell culture flask into a humidified incubator at 37 $\pm$ 1oC and 5.0 $\pm$ 1% $CO_2$;
- The cells are passaged every 3 to 4 days.

## Example 2

### Culturing of THP-1 cells

- Subcultures of THP-1 cells are maintained by re-suspending the cells in fresh culture medium;
- When the cells reach a cell density of $8.0 \times 10^5$ to $1.0 \times 10^6$ viable cells/ml, the cells need to be sub-cultured;
- Collect cells and spin down at 200g for 5 min. Resuspend the pellet in 10 ml with fresh pre-warmed RPMI-1640 + 10% FBS;
- Take a sample for cell counting;
- Dilute the cells to a density of $2.0 \times 10^5$ cell viable cells/ml with fresh pre-warmed RPMI-1640 + 10% FBS + L-Glu, and add 20 ml of the cell suspension to a new T-75 culture flask;
- Place the T-75 culture flask into a humidified incubator at 37 $\pm$ 1oC and 5.0 $\pm$ 1% $CO_2$;
- The cells are passaged every 3 and 4 days.

## Example 3

### Preparing and storing stock and working solutions

- Prepare LPS stock;
- LPS is stored at 2-8°C before reconstitution. To reconstitute, add 1ml sterile PBS to the LPS vial and gently swirl until the powder dissolves. The solution has a concentration of 1mg/ml. Aliquot the stock solution in 20x 50μl portions and store the vials at -80°C until further use;
- Prepare PMA stock;
- PMA is stored at -20°C before reconstitution. To reconstitute, add 1ml DMSO to the PMA vial by using a syringe and gently swirl until the powder is completely dissolved. Transfer the PMA solution to a 15 ml Falcon tube and add another 4ml DMSO. The solution has a concentration of 1mg/ml. Aliquot the stock solution in 49x 100 μl portions and 20x 5 μl portions. Store the vials at -80°C until further use;
- Prepare LPS/PMA Working Solutions;
- Thaw one LPS aliquot containing 50 μl with a concentration of 1 mg/ml and one PMA aliquot containing 5 μl with a concentration of 1 mg/ml;
- Take 40 μl from the LPS aliquot and dilute the solution with 360 μl RPMI-1640+10% FBS to a concentration of 100 μg/ml. This is the LPS Working stock;
- Add 10 ml RPMI-1640+10% FBS + 2 mM L-Glutamine to the 5 μl PMA aliquot, giving a concentration of 500 ng/ml. This is the PMA Working stock;
- Prepare the LPS/PMA working solution, by adding 10 μl of the LPS working stock and 400 μl of the PMA working stock to 9.6 ml RPMI-1640+10% FBS+ 2 mM L-Glutamine. Giving the combined solution a final concentration of 100 ng/ml LPS and 20 ng/ml PMA.

## Example 4

### Assay procedure

Seeding of the cell plate

- THP-1 cells are harvested and centrifugated at 200g for 5 minutes;
- Resuspend the pellet in 10 ml fresh pre-warmed RPMI-1640+10% FBS+2mM L-Glutamine;
- Take a sample for cell counting;
- After cell count adjust the cell density to 5 x 10⁵ cells/ml and add 100 μl of the THP-1 cell suspension to the wells of a 96-wellsplate (Corning Costar #3596);
- Fill the boundary wells of the plate with CGM and place the plate temporally into a humidified incubator at 37 $\pm$ 1oC and 5.0 $\pm$ 1% CO2.

Preparation of Complete Growth Medium (CGM)

- Heat-inactivated FBS (HI-FBS, 56°C, 45 min) is stored in 50 ml aliquots at -20°C;
- Pen/Strep (Gibco 15140) is stored in 5 ml aliquots at -20°C;
- RPMI 1640 (Gibco 21875) (500 ml bottles) is stored at 4°C as recommended by the provider;
- Make 500 ml of CGM by adding 50 ml of HI-FBS and 5 ml of Pen/Strep to 1 bottle of RPMI 1640;
- 100 μl of plasmocin (2.5 mg/ml, InVivoGen Cat #ant-mpp) are added to 50 ml aliquots of CGM and kept at 4°C

Treating

- In the dilution plate prepare the glatiramer acetate dilution series. The stock concentration is 20 mg/ml, which needs to be diluted in a 96-wellsplate to a working stock concentration of 1.2 mg/ml for the highest dilution on the dilution plate;

• Pipette 75 μl RPMI-1640+10% FBS in column 2-8 (row B-G) of the dilution plate and pipette 235 μl RPMI-1640+10% FBS in column 9 (row B-G). Note: Other wells of the dilution 96-wellsplate remain unused;

• Add 15 μl of reference glatiramer acetate and the test sample of glatiramer acetate (20 mg/ml) into column 9 (row B-G) of the dilution plate, giving a new concentration of 1.2 mg/ml;

• Serially dilute 150 μl down, from column 9 to 2. Mix at each step by pipetting up and down 5 times;

• Add 50 μl of the LPS/PMA working solution to the cell plate, with exception of the boundary wells.The final concentrations are 25 ng/ml LPS and 5 ng/ml PMA;

• Add 50 μl of the glatiramer acetate dilutions to the plate containing the cells and the LPS and PMA working solution. The final dose-range of glatiramer acetate is between 12.5 and 250 μg/ml;

• Incubate cells for 72 hours at 37°C in an incubator with 5% $CO_2$, 93% humidity.

<u>Supernatant harvest and ELISA</u>

• After 72 hours of incubation centrifuge the 96-well plate at 200g for 5 minutes.
• Transfer 150 μl of the cell-free supernatant/well to a storage plate.
• Supernatant of the samples will be used for analysis by IL-1Ra/Il-1F3 ELISA.
• sIL-1Ra is determined using the sIL-1Ra ELISA kit from Quantikine, R&D, Minneapolis, MN (Cat # DRAOOB) following the manufacturer's instructions. The remainder of the supernatant is stored in a closed storage plate at -80°C.

Example 5

<u>Data analysis</u>

Data on sIL-1Ra concentration are analyzed for relative potency as described in draft USP chapter <1034> section 3.4 on nonlinear models for quantitative responses. The software package GraphPad Prism (5.x) is used for fitting to a nonlinear model to the data:

• sIL-1Ra data (in pg/ml) are plotted against the log transformed glatiramer acetate concentration (in log μg/ml);
• A four-parameter logistic model is constructed to fit the dose response curves of the Reference (Ref) and the test batch (TA). To test if the dose response curves for the Reference and test batch are parallel (i.e. dose response curves of Reference and test are identical in shape but differ only in a constant horizontal difference) a likelihood ratio test may be used comparing the following two models: 1) separate models fitted for the reference and test batch for top, bottom, slope and EC50, 2) reduced model with common top, bottom and slope fitted to the Reference and test batch but with a different EC50. The four parameters in these fits are top and bottom asymptotes, slope, and EC50. A log likelihood ratio test is used to determine parallelism of results, since the reduced model (assuming parallelism) is contained in the full model (freely fitting both Ref and TA curves).

Upon confirmation of parallelism the reduced model will be fitted and the EC50 for the Reference and test batch will be estimated by EC50Ref and EC50TA respectively. The relative potency of the test batch will be estimated as:

$$\text{Relative potency} = EC50Ref/EC50TA * 100\%$$

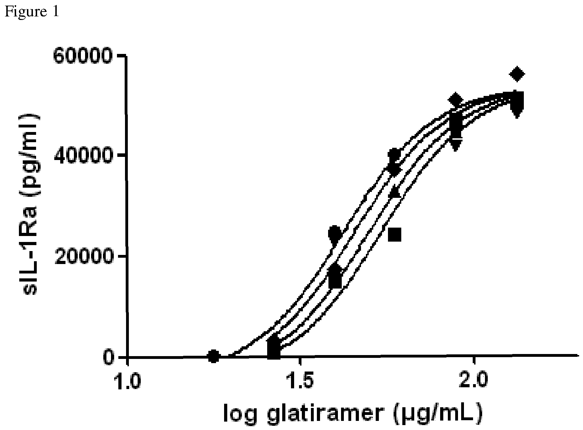

Figure 1. Four-parameter fits of sIL-1Ra response curves in THP-1 cells by increasing doses of glatiramer acetate (Synthon glatiramer acetate reference material). Cells were activated in medium containing 5 ng/ml PMA and 25 ng/ml LPS. The protocol was executed as described above. Four independent dose-response curves were generated of the same compound (100% test samples). Parallelism of results for the 4 fits was established as described in section 4.4. Top and bottom asymptotes, slope and EC50 were calculated. Goodness of fit ($R^2$) was between 0.97 and 0.99 for the 4 fits. Best-fit values and calculated relative potency for each curve are presented in Table 1. The range of relative potencies calculated for the four 100% samples is <30%, which is an indication of the precision of the method.

Table 1: Best-fit values of the constrained fits of the 4 response curves generated using identical Synthon glatiramer acetate reference material. Since each curve is generated from a 100% sample, one curve (curve A) has been arbitrarily picked and set at 100% relative potency. The other curves are treated as test articles and potencies are calculated relative to curve A.

| Best-fit values | curve A | curve B | curve C | curve D |
|---|---|---|---|---|
| Bottom | -4031 | -4031 | -4031 | -4031 |
| Top | 53602 | 53602 | 53602 | 53602 |
| LogEC50 | 1.624 | 1.733 | 1.698 | 1.656 |
| Slope | 3.306 | 3.306 | 3.306 | 3.306 |
| EC50 | 42.05 | 54.02 | 49.84 | 45.28 |
| Relative potency (%) | 100.0 | 128.5 | 118.5 | 107.7 |

The above data show that human monocytic cell lines can be used to assess the activity or potency of GA after stimulation of said cells by PMA and LPS. The parallelism of the four-parameter sigmoid dose-response curves in Fig. 1 indicate that the assay with sIL-1Ra as response is suitable to calculate the relative potency of batches against a Reference batch.

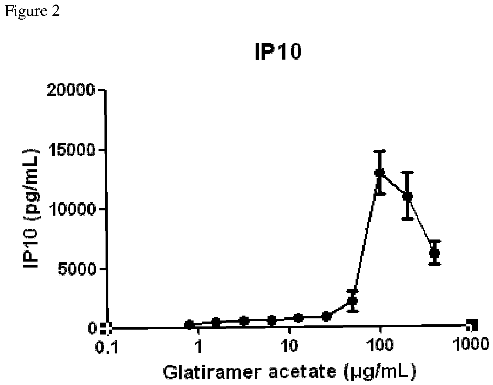

In Figure 2, the dose-response curve as derived from the data on IP-10 shown in Table 3 of WO 2008/157697 is depicted. As can be seen, this curve has a very steep slope, only a single point in the sloping part of the curve, and a pronounced hook effect at high dosages. From this single dose response curve it cannot be demonstrated that an assay with IP-10 as response is suitable for potency calculations. Similar results were obtained for the I-TAC and MIG data in Table 3.

**Claims**

1. A method to determine the potency of a batch of glatiramer acetate comprising stimulating human monocytic cell line cells with an effective amount of phorbol 12-myristate 13-acetate (PMA) and lipopolysaccharide (LPS), exposing said cells to said batch of glatiramer acetate (GA), and determining the expression of a monocyte anti-inflammatory cytokine induced by glatiramer acetate;

2. A method according to claim 1, **characterized in that** it further comprises determining the relative potency by comparing the potency of a batch of GA with the potency of a reference batch of GA;

3. A method according to claim 1 or 2, **characterized in that** GA is a copolymer consisting of the acetate salts of synthetic polypeptides made up of the amino acids tyrosine, glutamic acid, alanine, and lysine in all molar ratios;

4. A method according to any one of claims 1 to 3, **characterized in that** said human monocytic cells are selected from the group consisting of human acute monocytic leukemia cells (THP-1), human leukemic monocyte lymphoma cells (U937), and human monocytic leukemia cells Mono Mac 6 (MM6);

5. A method according to claim 4, **characterized in that** said cells are THP-1 cells;

6. A method according to any one of claims 1 to 5, **characterized in that** human monocytic cells are contacted with 0-250 µg/ml GA;

7. A method according to any one of claims 1 to 6, **characterized in that** human monocytic cells are stimulated with a combination of 5-10 ng/ml PMA and 5-25 ng/ml LPS;

8. A method according to any one of claims 1 to 7, **characterized in that** said monocyte anti-inflammatory cytokine is selected from the group consisting of secreted form of IL-1 receptor antagonist (sIL-1Ra), interleukin-10 (IL-10), and transforming growth factor beta (TGFβ);

9. A method according to claim 8, **characterized in that** said monocyte anti-inflammatory cytokine is sIL-1Ra;

10. A process for preparing a batch of glatiramer acetate (GA) which is acceptable for pharmaceutical use comprising a) preparing a batch of glatiramer acetate, b) measuring the relative potency of said batch according to the method of any one of claims 1 to 9, and c) qualifying the batch as acceptable for pharmaceutical use if the relative potency so measured is between 70% and 141% of a reference batch of glatiramer acetate;

11. A process according to claim 10, **characterized in that** said pharmaceutical use is for the treatment of multiple sclerosis (MS).

Figure 1

Figure 2

**IP10**

Europäisches
Patentamt

European
Patent Office

Office européen
des brevets

## EUROPEAN SEARCH REPORT

Application Number

EP 12 16 0140

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D<br><br>A | WO 2008/157697 A2 (MOMENTA PHARMACEUTICALS INC [US]; D ALESSANDRO JOSEPHINE S [US]; KISHI) 24 December 2008 (2008-12-24)<br>* the whole document *<br>* page 3, line 27 *<br>* page 4, lines 14-20 *<br>* pages 10-11 *<br>* claims 8,14,15 *<br>* table 1 * | 1-8,10, 11<br><br>9 | INV.<br>G01N33/50<br>G01N33/68 |
| Y<br><br><br><br><br><br><br><br>A | DANIELLE BURGER ET AL: "Glatiramer acetate increases IL-1 receptor antagonist but decreases T cell-induced IL-1 beta in human monocytes and multiple sclerosis", PROCEEDINGS OF THE NATIONAL ACADEMY OF SCIENCES OF THE UNITED STATES OF AMERICA, vol. 106, 1 January 2009 (2009-01-01), pages 4355-4359, XP55025401,<br>* the whole document *<br>* page 4358, column 2 * | 1-8,10, 11<br><br><br><br><br><br><br><br>9 | |
| A | WO 2009/129018 A1 (MOMENTA PHARMACEUTICALS INC [US]; ZHU XIANGPING [US]; SHRIVER ZACHARY) 22 October 2009 (2009-10-22)<br>* the whole document *<br>* claims 1,2,8,14 * | 1-11 | TECHNICAL FIELDS SEARCHED (IPC)<br><br>G01N |

The present search report has been drawn up for all claims

| Place of search | Date of completion of the search | Examiner |
|---|---|---|
| The Hague | 24 April 2012 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 642 290 A1

**ANNEX TO THE EUROPEAN SEARCH REPORT
ON EUROPEAN PATENT APPLICATION NO.**

EP 12 16 0140

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2012

| Patent document cited in search report | Publication date | Patent family member(s) | Publication date |
|---|---|---|---|
| WO 2008157697 A2 | 24-12-2008 | AU 2008265692 A1 | 24-12-2008 |
| | | CA 2690402 A1 | 24-12-2008 |
| | | CN 101743470 A | 16-06-2010 |
| | | EP 2174130 A2 | 14-04-2010 |
| | | EP 2381254 A1 | 26-10-2011 |
| | | JP 2010530975 A | 16-09-2010 |
| | | US 2011053203 A1 | 03-03-2011 |
| | | WO 2008157697 A2 | 24-12-2008 |
| WO 2009129018 A1 | 22-10-2009 | EP 2277050 A1 | 26-01-2011 |
| | | US 2009263347 A1 | 22-10-2009 |
| | | US 2010331266 A1 | 30-12-2010 |
| | | WO 2009129018 A1 | 22-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9531990 A
- US 3849550 A
- WO 03048735 A
- WO 2008157697 A

**Non-patent literature cited in the description**

- **D. BURGER et al.** *PNAS,* 2009, vol. 106 (11), 4355-4359